## Europäisches Patentamt

## European Patent Office

## Office européen des brevets

(11) Veröffentlichungsnummer: **0 078 469**
B1

(12) # EUROPÄISCHE PATENTSCHRIFT

(45) Veröffentlichungstag der Patentschrift:
09.04.86

(21) Anmeldenummer: 82109795.3

(22) Anmeldetag: 23.10.82

(51) Int. Cl.⁴: **A 61 K 31/505**, A 61 K 31/62 //
(A61K31/505, 31:18)

(54) **Neue pharmazeutische Zusammensetzung.**

(30) Priorität: 03.11.81 DE 3143471

(43) Veröffentlichungstag der Anmeldung:
11.05.83 Patentblatt 83/19

(45) Bekanntmachung des Hinweises auf die Patenterteilung:
09.04.86 Patentblatt 86/15

(84) Benannte Vertragsstaaten:
AT BE CH DE FR GB IT LI LU NL SE

(56) Entgegenhaltungen:
US - A - 3 081 230
US - A - 3 567 777

CHEMICAL ABSTRACTS, vol. 85, nr. 15, 11 Oktober 1976,
Seite 49, Zusammenfassung Nr. 104041k, COLUMBUS
OHIO (US).

Die Akte enthält technische Angaben, die nach dem
Eingang der Anmeldung eingereicht wurden und die
nicht in dieser Patentschrift enthalten sind.

(73) Patentinhaber: Beiersdorf Aktiengesellschaft,
Unnastrasse 48, D-2000 Hamburg 20 (DE)

(72) Erfinder: Dies, Rainer, Dr., Liebermannstrasse 48,
D-2000 Hamburg 52 (DE)
Erfinder: Asmussen, Bodo, Dr., Dorotheenweg 1a,
D-2071 Ammersbeck (DE)

# 0 078 469

## Beschreibung

Gegenstand der Erfindung ist eine neue pharmazeutische Zusammensetzung, ihre Verwendung und ihre Herstellung.

Das 4-Chlor-5-sulfamoyl-salicylsäure-(2,6-dimethyl-anilid) ist als Xipamid bekannt und als Diuretikum eingeführt, vgl. US-PS 3 567 777.

Das 2,4,7-Triamino-6-phenyl-pteridin ist als Triamteren bekannt und ein anerkanntes Diuretikum, vgl. US-PS 3 081 230.

Xipamid und Triamteren werden in der Humanmedizin seit vielen Jahren aufgrund ihrer diuretischen und antihypetensiven Eigenschaften verwendet.

Es ist auch bereits von Hohenegger/Holzer, (Int. J. Clin. Pharmacol. 13 1976, 289 - 303) eine Kombination von Xipamid und Triamteren bekannt, wobei die vorgesehenen Gewichtsverhältnisse von Xipamid zu Triamteren 1: 0,5, 1: 1 oder 1: 1,5 betragen. Dabei wird insbesondere dem Gewichtsverhältnis 1: 1 besondere Bedeutung beigemessen (Seite 302, rechte Spalte Mitte), demgegenüber das Verhälnis 1: 1,5 keinen weiteren Vorteil bietet.

Es wurde nun überraschend gefunden, daß eine pharmazeutische Zusammensetzung enthaltend 1 Gewichtsteil Xipamid und 3 - 4 Gewichtsteile Triamteren außerordentlich günstige pharmakologische und toxikologische Eigenschaften aufweist.

Xipamid ist ein Saluretikum von starker und mittellang andauern der Wirkung. Die diuretische und saluretische Wirkung erstreckt sich über einen Zeitraum von etwa 16 Stunden. Seine Verwendung als Monosubstanz führt zu Hypokaliämie, die als Ursache von Müdigkeit, Krämpfen und ähnlichen Beschwerden angesehen werden muß.

Triamteren ist ein Diuretikum, das die Natrium- und Wasserausscheidung erhöht, jedoch die Kaliumausscheidung verringert. Verwendet man Xipamid und Triamteren erfindungsgemäß im Gewichtsverhältnis 1: 3 bis 1: 4 zusammen, so vermag das Triamteren den kaliuretischen Effekt des Xipamids aufzuheben. Die Verwendung der erfindungsgemaßen Zusammensetzung erlaubt daher eine wirksame diuretische und antihypertensive Behandlung über einen längeren Zeitraum. Während der Behandlung wird dabei ein zufriedenstellendes hydroelektrisches Gleichgewicht des Blutes aufrechterhalten. In unerwarteter Weise wird bei dieser Behandlung bei gesicherter Natriurese und Wasserausscheidung die Kaliumausscheidung nicht erhöht. Dieser überragende pharmakologische Effekt war aus dem Stand der Technik nicht herleitbar.

Zudem wurde in nicht vorhersehbarer Weise gefunden, daß die Toxizität der erfindungsgemäßen Mischung geringer ist als diejenige, die man aufgrund der Toxizitäten der Bestandteile erwarten konnte. Bei der oralen Verabreichung an Mäusen ist die Toxizität der erfindungsgemäßen Zusammensetzung geringer als die Toxizität ihrer Bestandteile.

Damit ist die erfindungsgemäße pharmazeutische Zusammensetzung in der Humanmedizin insbesondere in der Therapie von Odemen unterschiedlicher Genese, bestimmten Formen der Adipositas, und ferner zur Behandlung refraktärer Ödeme, insbesondere bei Herzinsuffizienz, sowie in der Langzeitbehandlung der arteriellen Hypertonie geeignet.

Bevorzugt enthalten die erfindungsgemäßen pharmazeutischen Zusammensetzungen 1 Gewichtsteil Xipamid und 3 Gewichtsteile Triamteren. Beispielsweise enthält eine erfindungsgemäße Tablette oder ein Dragee 10 mg Xipamid, 30 mg Triamteren und 180 mg Exzipient.

Beim Erwachsenen verabreicht, kann die Tagesdosis der oral verabreichten Wirkstoffe beispielsweise etwa 10 bis 30 mg Xipamid und entsprechende Mengen Triamteren betragen, vorzugsweise etwa 10 mg Xipamid und 30 mg Triamteren bis etwa 20 mg Xipamid und 60 mg Triamteren.

Die erfindungsgemäßen pharmazeutischen Zusammensetzungen können parenteral oder über den Verdauungstrakt verabreicht werden, wobei die orale Verabreichung bevorzugt ist.

Diese pharmazeutischen Zusammensetzungen können beispielsweise fest oder flüssig sein und in den bei der Humanmedizin überilcherweise verwendeten pharmazeutischen Formen vorliegen, beispielsweise als Tabletten, Dragees, Gelkügelchen, Granulat, trinkbare Suspension, Suppositorien, injizierbare Lösung, deren Herstellung nach den üblichen Verfahren erfolgen kann. Zu den Wirkstoffen können die bei diesen pharmazeutischen Zusammensetzungen üblicherweise verwendeten Exzipienten hinzugefügt werden, beispielsweise Talkum, kolloidales Siliciumdioxid, Gummi arabicum, Lactose, Stärke, Magnesiumstearate, Kakaobutter, wässrige oder nicht wässrige Vehikel, Fette tierischen oder pflanzilchen Ursprungs, paraffinische Derivate, Glykole, verschiedene Netz-, Dispergier- oder Emulgiermittel und/oder Konservierungsmittel.

Das nachstehende Beispiel erläutert die Erfindung, ohne sie jedoch einzuschränken.

## Pharmazeutische Zusammensetzung

Man stellt Tabletten her, die der folgenden Formulierung entsprechen:

Xipamid 10 mg
Triamteren 30 mg
Exzipient q.s. 220 mg
Exzipient: Aerosil, Maisstärke, behandelte Stärke, Lactose, Magnesiumstearat, Talkum.

# 0 078 469

**Pharmakologische Untersuchung**

**1. Diuretische Aktivität:**

Es wurde die diuretische Aktivität der neuen erfindungsgemäßen Kombination und ihrer Bestandteile auf Diurese, Natriurese und Kaliurese an der normalen hydratisierten Ratte untersucht.

Die diuretischen Untersuchungen wurden an weiblichen Wistar Ratten (Ivanovas/Kisslegg (Allg.)). Im Gewicht von 180-200 g vorgenommen. Den Tieren war am Vorabend des Versuchstages das Trinkwasser entzogen worden. Standard-Rattendiät (Altromin 1324) stand ad ilbitum zur Verfügung. Bei Versuchbeginn erhielten die Tiere per Magensonde zur Hydratisierung je 5 ml einer 0,3-proh Kochsalzlösung verabreicht. Nach p.o. Appilkation der Prüfsubstanzen wurden die Tiere in Edelstahl-Stoffwechselkäfigen in einem klimatisierten Raum bei 22 ± 1°C und ca 65 % relativer Luftfeuchte gehalten.

Die Harnsammlung erfolgte über 6 Stunden in kailumfreien Behältern. Xipamid, Triamteren und Kombination wurden in den Dosen von 0,1 mg/kg (Xipamid) 0,3 mg/kg (Triamteren) und in der Kombination dieser Dosen an jeweils 25 Tieren verabreicht. Die Bestimmung der ausgescniedenen Elektrolyte Natrium und Kailum erfolgte flammenphotometrisch mit einem BECKMANN-Spektralphotometer. Die Ergebnisse sind in Tab. 1 angegeben.

**Tabelle 1:**

Übersicht über Harn und Elektrolytausscheidung nach Gabe von Xipamid und Triamteren und der Kombination ($\bar{x} \pm 2s\,\bar{x}$). Dosisverhältnis 1: 3.

| | Vol. ml/kg/6h | $\triangle$ % | $Na.^+$ mmol/kg/6h | $\triangle$ % | $K^+$ mmol/kg/6h | $\triangle$ % |
|---|---|---|---|---|---|---|
| ohne Behandlung | 24.3 $\pm$ 3.8 | | 1.00 $\pm$ 0.3 | | 1.60 $\pm$ 0.3 | |
| Xipamid 1.0 mg/kg | 47.4 $\pm$ 3.4 | +95.1 | 2.7 $\pm$ 0.3 | +170 | 2.7 $\pm$ 0.3 | +69 |
| Triamteren 3.0 mg/kg | 20.5 $\pm$ 2.4 | -15.6 | 1.0 $\pm$ 0.2 | $\pm$ 0 | 0.9 $\pm$ 0.2 | -44 |
| Xipamid/ Triamteren 1.0/3.0 mg/kg | 50.3 $\pm$ 3.4 | +107 | 3.7 $\pm$ 0.3 | +270 | 0.7 $\pm$ 0.2 | -56 |

Aus der vorstehenden Tabeile folgt, daß die neue erfindungsge-maße Kombination auf dem Gebiet der Diurese und der Natriurese eine ausgezeichnete Wirksamkeit sicherstellt, wobei im übrigen sehr bemerkenswert ist, daß diese Diurese und Natriurese von einer großen Einsparung der Kaliumausscheidung begleitet sind, da diese Ausscheidung in unerwarteter Weise viel geringer ist als sie aufgrund der Bestandteile erwartet werden

3

konnte.

## 2. Bestimmung der akuten Toxizität bei oraler Verabreichung an der Maus

Die akute Toxizität wurde an Gruppen von 10 männichen, nicht hydratisierten Mäusen von etwa 20 g bestimmt. Die Verbindungen wurden oral in 0,5% Methylcelluloseschleim mittels Ösophagussonde verabreicht. Xipamid und Triamteren wurden getrennt und in Mischung mit Triamteren verabreicht. Es wurden folgende Letailtäten ermittelt:

### 2.1 Xipamid

| Dosis mg/kg KG *) | Letalität |
|---|---|
| 0 | 0/10 |
| 631 | 2/10 |
| 1000 | 6/10 |

$LD_{50}$ (95% Vertrauensbereich) 877 mg/kg
*) KG = Körpergewicht

### 2.2 Triamteren

| Dosis mg / kg KG | Letalität |
|---|---|
| 0 | 0/10 |
| 500 | 3/10 |
| 2000 | 6/10 |

$LD_{50}$ (95% Vertrauensbereich) 1725 mg/kg

### 2.3 Kombination

für die Ermittlung der Toxizität der Komälnation wurden nicht hydratisierte C.D. Mäuse verwendet. Es wurden folgende Letailtäten registriert:

| Xipamid (mg/kg) | Triamteren (500 mg/kg) |
|---|---|
| 0 | 3/10 |
| 1500 | Wert niedriger als |
| | Summe der Einzelwerte |

## Patentansprüche für die Vertragsstaaten BE CH DE FR GB IT LI LU NL SE

1. Pharmazeutische Zusammensetzung, enthaltend 1 Gew.-Teil Xipamid (4-Chlor-5-sulfamoyl-salicylsäure-(2,6-dimethyl-anilid)) und 3 - 4 Gew.-Teile Triamteren (2, 4, 7-Triamino-6-phenyl-pteridin).

2. Pharmazeutische Zusammensetzung nach Anspruch 1, enthaltend 1 Gew. Teil Xipamid und 3 Gew. Teile Triamteren.

3. Pharmazeutische Zusammensetzung nach einem der Ansprüche 1 oder 2, enthaltend für die orale Verabreichung geeignete pharmazeutische Trägersubstanzen.

4. Pharmazeutische Zusammensetzung nach einem der Ansprüche 1 - 3 in Form von Tabletten oder Dragees, enthaltend 10 mg Xipamid und 30 mg Triamteren.

5. Pharmazeutische Zusammensetzung nach einem der Ansprüche 1 - 4 zur diuretischen und antihypertensiven Behandlung.

6. Verfahren zur Herstellung der pharmazeutischen Zusammensetzung nach einem der Ansprüche 1 - 4, dadurch gekennzeichnet, daß man 1 Gew.-Teil Xipamid mit 3 - 4 Gew.-Teilen Triamteren gegebenenfalls zusammen mit einem pharmazeutischen Träger zu einem pharmazeutischen Präparat verarbeitet.

**0 078 469**

**Patentansprüche** für den Vertragsstaat AT

1. Pharmazeutische Zusammensetzung, enthaltend 1 Gew -Teil Xipamid (4-Chlor-5-sulfamoyl-salicylsäure-(2,6-dimethyl-anilid)) und 3 - 4 Gew.-Teile Triamteren (2,4,7-Triamino-6-phenyl-pteridin).

2. Pharmazeutische Zusammensetzung nach Anspruch I, enthaltend 1 Gew.-Teil Xipamid und 3 Gew.-Teile Triamteren.

3. Pharmazeutische Zusammensetzung nach einem der Ansprüche 1 oder 2, enthaltend für die orale Verabreichung geeignete pharmazeutische Trägersubstanzen.

4. Pharmazeutische Zusammensetzung nach einem der Ansprüche I - 3 in Form von Tabletten oder Dragees, enthaltend 10 mg Xipamid und 30 mg Triamteren.

5. Pharmazeutische Zusammensetzung nach einem der Ansprüche 1 - 4 zur diuretischen und antihypertensiven Behandlung.

6. Verfahren zur Herstellung einer pharmazeutischen Zusammensetzung, enthaltend 1 Gew.-Teil Xipamid und 3 - 4 Gew.-Teile Triamteren, dadurch gekennzeichnet, daß man 1 Gew.-Teil Xipamid mit 3 - 4 Gew.-Teilen Triamteren gewünschtenfalls zusammen mit einem pharmazeutischen Träger zu einem pharmazeutischen Präparat verarbeitet.

7. Verfahren zur Herstellung der pharmazeutischen Zusammensetzung nach Anspruch 6, dadurch gekennzeichnet, daß man 1 Gew.-Teil Xipamid mit 3 Gew.-Teilen Triamteren gewünschtenfalls zusammen mit einem pharmazeutischen Träger zu einem pharmazeutischen Präparat verarbeitet.

8. Verfahren zur Herstellung einer pharmazeutischen Zusammensetzung nach Anspruch 6 in Form von Tabletten oder Dragees, enthaltend 10 mg Xipamid und 30 mg Triamteren.


**Claims** for the contracting states BE, CH, DE, FR, GB, IT, LI, LU, NL, SE.

1. Pharmaceutical composition containing 1 part by weight of xipamide (4-chloro-5-sulfamoylsalicylyl-(2,6-dimethylanilide)) and 3 - 4 parts by weight of triamterene (2,4,7-triamino-6-phenylpteridine).

2. Pharmaceutical composition according to Claim 1, containing 1 part by weight of xipamide and 3 parts by weight of triamterene.

3. Pharmaceutical composition according to one of Claims 1 or 2, containing pharmaceutical vehicles suitable for oral administration.

4. Pharmaceutical composition according to one of Claims 1 - 3, in the form of tablets or coated tablets containing 10 mg of xipamide and 30 mg of triamterene.

5. Pharmaceutical composition according to one of Claims 1 - 4, for diuretic and antihypertensive treatment.

6. Process for the preparation of the pharmaceutical composition according to one of Claims 1 - 4, characterised in that 1 part by weight of xipamide is processed together with 3 - 4 parts by weight of triamterene, where appropriate together with a pharmaceutical vehicle, to give a pharmaceutical product.


**Claims** for the contracting state AT

Pharmaceutical composition containing 1 part by weight of xipamide (4-chloro-5-sulfamoylsalicylyl-(2,6-dimethylanilide)) and 3 - 4 parts by weight of triamterene (2,4,7-triamino-6-phenylpteridine).

2. Pharmaceutical composition according to Claim 1, containing 1 part by weight of xipamide and 3 parts byweight of triamterene.

3. Pharmaceutical composition according to one of Claims 1 or 2, containing pharmaceutical vehicles suitable for oral administration.

4. Pharmaceutical composition according to one of Claims 1 - 3, in the form of tablets or coated tablets containing 10 mg of xipamide and 30 mg of triamterena.

5. Pharmaceutical composition according to one of Claims 1 - 4, for diuretic and antihypertensive treatment.

6. Process for the preparation of a pharmaceutical composition containing 1 part by weight of xipamide and 3 - 4 parts by weight of triamterene, characterised in that 1 part by weight of xipamide is processed together with 3 - 4 parts by weight of triamterene, if desired together with a pharmaceutical vehicle, to give a pharmaceutical product.

7. Process for the preparation of the pharmaceutical composition according to Claim 6, characterised in that 1 part by weight of xipamide is processed together with 3 parts by weight of triamterene, if desired together with pharmaceutical vehicle, to give a pharmaceutical product.

8. Process for the preparation of a pharmaceutical composition according to Claim 6, in the form of tablets or coated tablets containing 10 mg of xipamide and 30 mg of triamterene.

5

**Revendications** pour les Etats Contractants BE CH DE FR GB IT LI LU NL SE

1. Composition pharmaceutique, contenant une partie en poids de Xipamide (chloro-4-sulfamoyl-5-diméthyl-2',6'-salicy-lanilide) et 3-4 parties en poids de Triamtèrène (Triamino-2,4,7-phènyl-6-ptéridine).

2. Composition pharmaceutique selon la revendication 1, contenant 1 partie en poids de Xipamide et 3 parties en poids de Triamtérène.

3. Composition pharmaceutique selon l'une quelconque des revendications 1 ou 2, contenant des substancessupports pharmaceutiques appropriées à l'administration orale.

4. Composition pharmaceutique selon l'une quelconque des revendications 1 a 3 sous forme de comprimés ou de dragées, contenant 10 mg de Xipamide et 30 mg de Triamtérène.

5. Composition pharmaceutique selon l'une quelconque des revendications 1 a 4 pour le traitement diuretique et anti-hypertensif.

6. Procédé de préparation de la composition pharmaceutique selon l'une quelconque des revendications 1 à 4, caractérisé en ce que l'on façonne 1 partie en poids de Xipamide avec 3 à 4 parties en poids de Triamtérène, le cas échéant conjointement avec un support pharmaceutique, en une préparation pharmaceutique.

**Revendications** pour l'Etat Contractant AT

1. Composition Pharmaceutique, contenant une partie en poids de Xipamide (chloro-4-sulfamoyl-5-diméthyl-2',6'-salicylanilide) et 3 a 4 parties en poids de Triamtérène (Triamino-2,4,7-phenyl-6-ptéridine).

2. Composition pharmaceutique selon la revendication 1 contenant une partie en poids de Xipamide et 3 parties en poids de Triamtérène.

3. Composition pharmaceutique selon l'une quelconque des revendications 1 ou 2, contenant des substancessupports pharmaceutiques appropriées a l'administration orale.

4. Composition pharmaceutique selon l'une quelconque des revendications 1 a 3 sous forme de comprimés ou de dragées, contenant 10 mg de Xipamide et 30 mg de Triamtérène.

5. Composition pharmaceutique selon l'une quelconque des revendications 1 a 4 pour le traitement diuretique et anti-hypertensif.

6. Procédé de préparation d'une composition pharmaceutique, contenant 1 partie en poids de Xipamide et 3 a 4 parties en poids de Triamtérène, caractérisé en ce que l'on façonne 1 partie en poids de Xipamide avec 3 à 4 parties en poids de Triamtérène, si desire conjointement avec un support pharmaceutique, en une préparation pharmaceutique.

7. Procédé de préparation de la composition pharmaceutique selon la revendication 6 caractérisé en ce que l'on façonne 1 partie en poids de Xipamide avec 3 parties en poids de Triamtérène, si désiré conjointement, avecun support pharmaceutique en une préparation pharmaceutique.

8. Procédé de préparation d'une composition pharmaceutique selon la revendication 6 sous forme de comprimés ou de dragées, contenant 10 mg de Xipamide et 30 mg de Triamtérène.